(19) Europäisches Patentamt / European Patent Office / Office européen des brevets

(11) **EP 2 999 520 B1**

(12) **EUROPEAN PATENT SPECIFICATION**

(45) Date of publication and mention of the grant of the patent:
**15.08.2018 Bulletin 2018/33**

(51) Int Cl.:
*A61P 17/00* (2006.01)     *A61K 36/537* (2006.01)

(21) Application number: **14733334.8**

(22) Date of filing: **22.05.2014**

(86) International application number:
**PCT/IB2014/061625**

(87) International publication number:
**WO 2014/188370 (27.11.2014 Gazette 2014/48)**

(54) **NEW FRACTION OF SAGE EXTRACT AND USES THEREOF**

NEUE FRAKTION EINES SALBEIEXTRAKTS UND VERWENDUNGEN DAVON

NOUVELLE FRACTION D'EXTRAIT DE SAUGE ET UTILISATIONS ASSOCIÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.05.2013 IT RM20130299**

(43) Date of publication of application:
**30.03.2016 Bulletin 2016/13**

(73) Proprietor: **Aboca S.p.A. Società Agricola**
**52037 Sansepolcro (AR) (IT)**

(72) Inventor: **MERCATI, Valentino**
**I-52037 Sansepolcro AR (IT)**

(74) Representative: **Predazzi, Valentina et al**
**Società Italiana Brevetti S.p.A.**
**Piazza di Pietra, 39**
**00186 Roma (IT)**

(56) References cited:
**WO-A1-2012/065651     US-A1- 2004 208 902**
**US-A1- 2004 219 124**

• **Bioesti: "Sage (Salvia Officinalis)", , 17 May 2012 (2012-05-17), XP002703559, Retrieved from the Internet: URL:http://www.bioesti.com/index.php/sage-salvia-officinalis [retrieved on 2013-07-17]**
• **WECKESSER ET AL: "Screening of plant extracts for antimicrobial activity against bacteria and yeasts with dermatological relevance", PHYTOMEDICINE, GUSTAV FISCHER VERLAG, STUTTGART, DE, vol. 14, no. 7-8, 19 July 2007 (2007-07-19), pages 508-516, XP022163612, ISSN: 0944-7113, DOI: 10.1016/J.PHYMED.2006.12.013**
• **ANDREW B SCHOLEY ET AL: "An extract of Salvia (sage) with anticholinesterase properties improves memory and attention in healthy older volunteers", PSYCHOPHARMACOLOGY, SPRINGER, BERLIN, DE, vol. 198, no. 1, 19 March 2008 (2008-03-19), pages 127-139, XP019621050, ISSN: 1432-2072**
• **CSABA N ET AL: "The performance of nanocarriers for transmucosal drug delivery", EXPERT OPINION ON DRUG DELIVERY, INFORMA HEALTHCARE, GB, vol. 3, no. 4, 1 July 2006 (2006-07-01), pages 463-478, XP009171310, ISSN: 1742-5247**
• **DIRK LACHENMEIER ET AL: "Holistic Control of Herbal Teas and Tinctures Based on Sage (Salvia officinalis L.) for Compounds with Beneficial and Adverse Effects using NMR Spectroscopy", ANALYTICAL CHEMISTRY INSIGHTS, vol. 7, 1 January 2012 (2012-01-01), pages 1-12, XP055131448, ISSN: 1177-3901, DOI: 10.4137/ACI.S8946**

**Description**

TECHNICAL FIELD OF THE INVENTION

The present disclosure relates to a new fraction of sage extract in which only traces of rosmarinic acid, carnosic acid and derivatives thereof (e.g., carnosol), thujones and resins are present, a process for the preparation of such extract fraction, compositions comprising it, and the use of the extract fraction and of the compositions for the protection of the skin or mucous membranes.

STATE OF THE PRIOR ART

*Salvia officinalis* is an evergreen shrub belonging to the lamiaceae family and is native to the Mediterranean region.

This plant contains an essential oil to which antiinflammatory and antimicrobial properties are attributed and is used in the treatment of inflammations and infections of the mouth and throat, such as stomatitides, gingivitides and pharyngitides; also its indication in hyperhidrosis is interesting.

The dried leaves of the plant are commonly used.

Sage leaf consists in the dried leaves, whole or cut, of *Salvia officinalis* L. Whole spice contains not less than 1.3% of essential oil, and cut spice not less than 0.9% of essential oil, both calculated with reference to the dry spice.

*Salvia officinalis* contains resin, essential oil, hydroxycinnamic derivatives, with rosmarinic acid being the most representative one, flavonoids, terpene phenols, where carnosic acid and carnosol are among the most representative ones, polysaccharides, tannins.

Rosmarinic acid, together with carnosic acid and its derivatives, like, e.g., carnosol, are deemed the main active principles accountable for the antiinflammatory and antimicrobial properties of *Salvia officinalis.*

Thujone (alpha and beta) is instead a component very abundant in sage essential oil (even over 50% in weight) which has toxic activities and a convulsant effect.

In particular, in the literature reference is made to a potential interaction danger in the administration of sage with CNS anticonvulsants and sedatives.

Sage leaves generally contain up to 3% of essential oil.

Sage extract, obtained by extraction with hydroalcoholic mixtures, after drying contains about 3% in weight of rosmarinic acid, about 2% in weight of carnosic acid and total derivatives thereof and about 0.3% in weight of alpha and beta thujone. Therefore, the hydroalcoholic extraction and the process of drying by lyophilization (for extracts dried by lyophilization) strongly reduce thujones' presence, while carnosic acid and derivatives thereof and rosmarinic acid, together representing about the 5% in weight of the dried hydroalcoholic extract, remain in a good percentage. The various known extraction processes are focused on improving the yield in known active principles or making such components more useful for therapeutic purposes.

SUMMARY OF THE INVENTION

The present invention is defined in claims 1-13.

The present disclosure relates to a sage extract fraction substantially depleted in the known pharmacologically active components represented by rosmarinic acid and carnosic acid and derivatives thereof (carnosol) and in thujones, of which only traces remain, and depleted also in resins, and to a process for obtaining such extract fraction. The extract fraction according to the disclosure may be used in a way completely different from that in which the initial sage extract is used in the state of the art, i.e., as mucous membranes or skin protective agent of the mucous membranes or of the skin on the basis of its mucoadhesive properties and of barrier effect (a property very low or substantially absent in the initial extract). Such use can be carried out thanks to the protective activities of the remaining components, in which the extract is enriched by 15-75% with respect to the initial product thanks to the removal of oleoresin and of rosmarinic acid, of carnosic acid and derivatives thereof and of the thujones therefrom. The extract fraction of the invention therefore provides a new source of material of plant origin useful for the protection of the mucous membranes and of the skin, wherein the pharmacological effects attributable to the components known as active principles are substantially reduced or eliminated.

Said condition is particularly desirable when materials of natural or plant origin are to be used for actions of a mainly mechanical type (i.e., not for pharmacological actions having, e.g., a direct effect on the activation of receptorial pathways, immune pathways or metabolic processes). The use of substances with a mainly mechanical effect, like e.g. substances having a barrier or mucoadhesive effect, is desirable, e.g., for assisting a pharmacological therapy. By minimizing the presence of substances with pharmacological activities in the material of natural or plant origin as in the present invention, a material with a mainly mechanical effect is obtained, which can be used in combination with drugs, minimizing possible undesired interactions with drugs and, at the same time, exploiting the typical feature of substances of plant origin, of having a vast number of components able to exert a protective effect besides the pharmacologically active ones commonly used. In the present invention, instead of using the plants for their pharmacologically active substances, a substantial elimination of the latter from the preparation is carried out, and fractions are used in which substances having a mechanical protective effect prevail, thereby obtaining compositions that can be used as protective agents of the skin or of the mucous membranes, of natural origin and that can be more easily associated with pharmacological therapies, since possible interactions with drugs are extremely reduced, given the depletion in active principles performed on the natural extracts. For sage, e.g., the potential danger of sage interaction with CNS anticonvulsants and sedatives is reported in the literature.

The use of fractions of extracts of plant or natural complex substances depleted in pharmacologically active principles entails the dual advantage of providing a material having a mainly mechanical protective action (mucous membrane protection or skin protection) and of enabling a concrete and useful use of materials normally discarded in extraction processes of plant or natural active principles, as the extraction processes as a rule aim at the greatest possible concentration/purification of the pharmacologically active principles.

The present invention therefore relates to a sage extract fraction depleted in rosmarinic acid, carnosic acid and derivatives thereof (carnosol) and thujones, wherein said rosmarinic acid has a concentration in weight percentage of at least $\leq 3\%$, said carnosic acid and carnosol have a concentration in weight percentage of $\leq 0.1\%$ and said thujones are in a percentage lower than or equal to 0.001% characterized in that it has a barrier effect greater than that of a non-fractioned hydroalcoholic extract of sage leaves; its use in the treatment or prevention of skin lesions which do not involve the presence of open wounds; compositions comprising such extract; their use in the treatment or prevention of skin lesions which do not involve the presence of open wounds and processes for the preparation of such extract and of such compositions.

The invention also relates to a process for the preparation of the sage extract fraction as defined above, comprising the following steps:

a. preparing a hydroalcoholic extract of sage leaves

b. obtaining from the extract prepared in a. a concentrated aqueous extract

c. decanting and/or filtering said aqueous extract collecting the supernatant or the filtrate wherein said supernatant or filtrate comprises rosmarinic acid at a concentration in weight percentage of $\leq 3\%$ and carnosic acid and carnosol at a concentration in weight percentage of $\leq 0.6\%$ and said thujones are in a percentage lower than or equal to 0.001%,

d. the supernatant or filtrate obtained in c. is subjected to one or more ultrafiltration steps and/or to one or more steps (passages) through a high porosity adsorbing resin and the fraction recovered after drying comprises rosmarinic acid at a concentration in weight percentage of $\leq 0.7\%$ and carnosic acid and carnosol at a concentration in weight percentage of $\leq 0.1\%$ and said thujones are in a percentage lower than or equal to 0.001% and

wherein said hydroalcoholic extract of step a. is prepared by subjecting sage leaves, optionally fragmented, to two or more extraction steps in ethanol at decreasing concentration ranging from 96% ethanol to 0% ethanol.

The percentage values are referred to the extract object of the invention after drying treatment by lyophilization.

DETAILED DESCRIPTION OF THE FIGURES

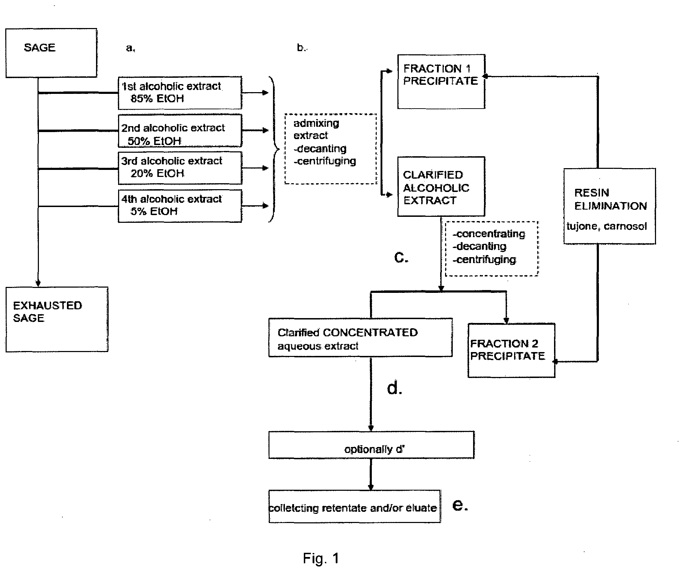

Figure 1 shows a block diagram of an embodiment of the process of the invention in which step d is not present.

Figure 2a shows a block diagram of step d.

Figure 3A shows a graph of mucoadhesion on an inclined plane (assay described below) in which the assayed substance is water.

Squares denote results obtained on an inclined plane with mucin and water, whereas rhombs denote those obtained on the control (blank) in which water is assayed on an inclined plane without mucin.

The figure shows that for water, a non-mucoadhesive substance, the measurements of the sliding on the inclined plane with and without mucin are practically overlappable.

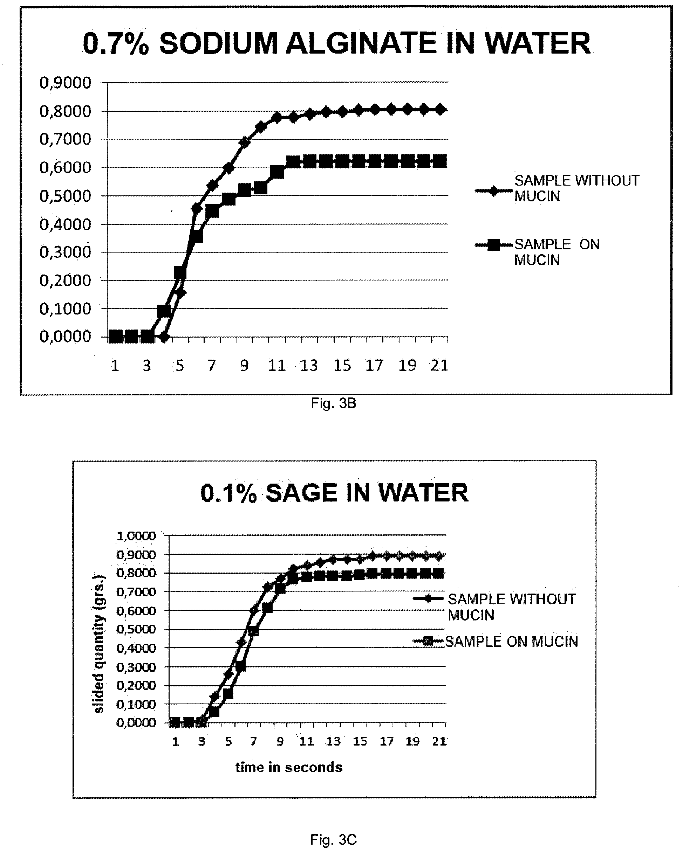

Figure 3B shows a graph of mucoadhesion on an inclined plane (assay described below) in which the assayed substance is sodium alginate, a substance with known mucoadhesive properties.

Squares denote results obtained on an inclined plane with mucin and sodium alginate, whereas rhombs denote those obtained on the control (blank) in which sodium alginate is assayed on an inclined plane without mucin.

In the case of the sample consisting of sodium alginate, a remarkable difference in behavior is witnessed in the presence and in the absence of mucin.

Figure 3C shows a graph of mucoadhesion on an inclined plane (assay described below) in which the assayed substance is the sage extract fraction according to the present description in order to verify its mucoadhesive properties.

Squares denote results obtained on an inclined plane with mucin and sage extract according to the present description, whereas rhombs denote those obtained on the control (blank) in which the sage extract according to the present description is assayed on an inclined plane without mucin.

As it may be observed, the extract fraction has a behavior similar to sodium alginate; a remarkable difference in behavior is witnessed in the presence and in the absence of mucin, denoting mucoadhesive features.

SHORT GLOSSARY

NMWCO=Nominal Molecular Weight Cut-off

PERMEATE: extract that has passed through the semi-permeable ultrafiltration membrane
RETENTATE: extract that did not pass through the ultrafiltration membrane or material adsorbed on resin.
ELUATE: material not adsorbed on resin following adsorption passages thereon.
ULTRAFILTRATION: filtration technique on semi-permeable membrane characterized by pores having sizes of 100,000 daltons (0.1 $\mu$m) to 500 daltons (about 0.005 $\mu$m)

SAGE PARTS USED: leaves

By "NOT GREATER THAN", relative to the amounts of active principles in the extract, in the present description it is meant ≤.

By "SUBSTANTIALLY ABSENT", with regards to thujones or even to the carnosic acid and derivatives thereof (carnosol) present in the extract of the invention, it is meant that the measurable percentage of such molecules in the extract is lower than or equal to 0.001%.

By initial or reference hydroalcoholic extract it is meant a non-fractioned hydroalcoholic extract of sage leaves as commonly used in the state of the art.

DETAILED DESCRIPTION OF THE INVENTION

As indicated above, in the present description a sage extract fraction depleted in rosmarinic acid, carnosic acid and derivatives thereof and thujones is provided, wherein said rosmarinic acid has a concentration in weight percentage ≤ 1% and said carnosic acid and derivatives thereof have a concentration in weight percentage of ≤0.6% and said thujones are in a percentage lower than or equal to 0.001%. By applying the extraction process object of the invention, described below, and separation techniques on semi-permeable membrane or on resin, it was possible to obtain a sage extract fraction depleted in resins, rosmarinic acid, carnosic acid and carnosol and thujones, and concomitantly surprisingly enriched in the remaining substances, which showed an effective protective action on the skin and on the mucous membranes, as evident from the mucoadhesivity assay and barrier assay reported in the experimental section below.

In particular, the extract fraction according to the invention exhibits a barrier effect greater than 10% or even than 20% compared to the initial extract, from which the fraction is obtained as it may be seen from the data reported below.

Compared to the common fluid, dry and/or lyophilized extracts of sage, and to sage as such, which comprise an amount of rosmarinic acid of at least about 3% in weight, of carnosol of about 2% in weight and of thujones of about 0.3%, the percentage values being referred to dried extracts of sage, the extract fraction of the present invention has merely traces of such active principles, as comprising an amount of rosmarinic acid of at least about ≤ 1% in weight and carnosic acid and carnosol of at least ≤ 0.6% in weight and thujones in a percentage lower than or equal to 0.001%, wherein said amounts in weight could also be an amount of about ≤0.7% of rosmarinic acid and an amount of ≤0.1 % or even of ≤0.05%, or even non-measurable, of carnosic acid and carnosol, thujones in a percentage lower than or equal to 0.001%.

Evidently, such percentages are, in practice, traces of rosmarinic acid, carnosic acid and derivatives thereof and of thujones that cannot induce the pharmacological activities (and the interactions with drugs related to such substances) for which sage or sage extracts are commonly used and traces of thujones depriving the extract fraction of the invention of the toxicity normally present in sage or sage extracts.

Since the extract fraction is parallelly depleted also in resins, the latter being them also almost completely eliminated from the extract in the same steps of elimination of rosmarinic acid, carnosic acid and derivatives thereof and thujones, the permanence of the mucoadhesive effect and the significant increase in the barrier effect observed for the fraction of the invention are surprising.

In particular, the extract of the present invention, has a barrier effect 10% or even 20% greater than the reference (also referred to as initial) extract from which it derives, and however keeps a significant mucoadhesion.

In fact, the comparative data obtained by the authors, reported in the section detailed below, show that the fraction of the invention has a relevant percentage of mucoadhesion in assay on an inclined plane and also shows a significant increase in barrier effect compared to the initial extract.

The extract fraction of the disclosure is therefore useful for its mucoadhesion and barrier effect properties in all those cases in which the protection of skin or mucous membranes is needed or desirable; those may be cases in which a drug that attacks mucous membranes or skin has to be administered, therefore in order to prevent the damaging action of the drug, or in those cases in which the protection of a partially compromised skin or mucous membrane is preferable or desirable so as to enable a better and quicker healing thereof, defending it from further aggressions, or in those cases in which an individual has a chronic disorder in which skin or mucous membranes sustain irritations or alterations, therefore a barrier effect can prevent or limit damages on the skin or the mucous membrane.

In outlining the protection effect of mucous membranes, in the present description it is meant that said mucous membranes can be the oral mucosa, gastric mucosa, intestinal mucosa, the nasal mucosa, the vaginal mucosa, the uterine mucosa, the rectal mucosa.

The present invention also provides a process for the preparation of the extract fraction as described above, said process comprising the following steps:

    a. preparing a hydroalcoholic extract of sage leaves

    b. obtaining from the extract prepared in a. a concentrated aqueous extract

    c. decanting and/or filtering said aqueous extract collecting the supernatant or the filtrate wherein said supernatant or filtrate comprises rosmarinic acid at a concentration in weight percentage of ≤3% and carnosic acid and carnosol at a concentration in weight percentage of ≤0.6% and said thujones are in a percentage lower than or equal to 0.001%,

    d. the supernatant or filtrate obtained in c. is subjected to one or more passages through a high porosity adsorbing resin and the collected fraction comprises rosmarinic acid at a concentration in weight percentage of ≤0.7% and carnosic acid and carnosol at a concentration in weight percentage of ≤0.1% and said thujones are in a percentage lower than or equal to 0.001% and

wherein said hydroalcoholic extract of step a. is prepared by subjecting sage leaves, optionally fragmented, to two or more extraction steps in ethanol at decreasing concentration ranging from 96% ethanol to 0% ethanol.

The process described herein could be carried out starting from sage leaves or fresh or dried parts thereof.

According to the invention, the above-described process can be carried out by performing at point a. at least two, at least three, or at least four steps in decreasing ethanol concentrations, in which the decreasing ethanol concentrations ranges from 96% ethanol to 0% ethanol (e.g. 0.5%).

For instance, step a. of the process may be carried out by performing a step in 85% ethanol, a step in 50% ethanol, and a step in 20% ethanol.

Or, step a. of the process could be carried out by performing a step in 85% ethanol, a step in 50% ethanol, a step in 20% ethanol and a step in 5% ethanol.

The extracts obtained as described above are gathered to form a hydroalcoholic extract. These can be gathered, for instance, in equal mutual ratios, as 50:50 in the case of two steps in ethanol, or as 33.3:33.3:33.3 in the case of three steps in ethanol or 25:25:25:25 in the case of four steps in ethanol. Evidently, the extracts obtained by performing the various above-described steps in ethanol could be gathered in different ratios, according to the general knowledge of the technician in the field.

As already indicated above, the hydroalcoholic extract obtained at step a. will be used to prepare a concentrated aqueous extract at step b.

Said concentrated aqueous extract could be prepared by concentrating the hydroalcoholic extract obtained in a. by alcohol evaporation and it is decanted and/or centrifuged and/or filtered and the aqueous concentrated phase is collected (for instance, in the case of decantation and/or centrifugation) the aqueous supernatant is collected, whereas in the case of filtration the filtrate is collected. Alternatively, alcohol evaporation could be performed after the centrifugation and/or decantation and/or filtration stage.

Decantation could be performed, e.g., 1 to 72 hours, and could be followed or replaced by one or more centrifugations to 3000-4000 (e.g., about 3500) rpm for a period comprised between 1 and 10 minutes, e.g. about 5 minutes. For filtration, suitable filters known to the technician in the field could be used, like, e.g., panel filters with a cutoff of about 25 micrometers.

The supernatant obtained after this step(s) is the collected and subjected, in case not already done before, to alcohol evaporation by standard techniques, like, e.g., by use of a thin film distillation system, or a batch concentration system according to standard protocols commonly used by the technician in the field.

The result of this evaporation is a concentrated aqueous extract that, in step c. of the process, is subjected to decantation and/or filtered.

The concentrated aqueous extract (which could also be referred to as concentrated aqueous solution) may be subjected to decantation for a period comprised between 1 and 72 hours, and the supernatant is then recovered, thereby obtaining a clarified or clear solution.

Decantation may be followed or replaced by one or more centrifugations at 3000-4000 (e.g., about 3500) rpm for a period comprised between 1 and 10 minutes, e.g. about 5 minutes.

Alternatively, the concentrated aqueous extract is filtered, thereby obtaining a clarified solution. Filtration could be performed, e.g., on a panel filter with a cutoff from 10 to 50 micrometers, like, e.g., a cutoff of 15-20 micrometers, or a cutoff of 25 micrometers or even higher.

In a further embodiment the concentrated aqueous extract could be decanted as described above and the supernatant obtained could then be filtered as described above.

The supernatant or the filtrate obtained as described above are recovered and constitute, after drying, a sage extract comprising rosmarinic acid at a concentration in weight not greater than 3% (i.e., ≤3%) and carnosic acid and carnosol at a concentration in weight not greater than 0.6% (i.e., ≤ 0.6%) and thujones in a percentage lower than or equal to 0.001%. The process of the invention comprises a further step d. of depletion in undesired substances from the supernatant or the filtrate obtained in the above-described step c. before collection of the desired extract fraction , wherein said supernatant or filtrate is subjected to one or more further depletion steps in rosmarinic acid, carnosic acid and derivatives thereof.

Such further step enables to obtain a sage extract fraction comprising rosmarinic acid at a concentration in weight percentage of ≤0.7% and carnosic acid and derivatives thereof at a concentration in weight percentage of ≤0.1%, more particularly this step enables to obtain an extract fraction in which thujones and carnosic acid and derivatives thereof are substantially absent.

This step d) is carried out by passage through adsorbing resin of the supernatant or filtrate obtained in c.

According to one embodiment, the process of the invention comprises a step d. wherein the filtrate or the supernatant obtained in c. is subjected to one or more passages through a high porosity adsorbing resin and the eluate is collected, wherein said eluate thus obtained comprises rosmarinic acid at a concentration in weight percentage of ≤0.7%, carnosic acid and carnosol at a concentration in weight percentage of ≤0.1% and thujones in a percentage lower than or equal to 0.001%.

In particular, the resin suitable to implement the invention is a resin able to adsorb aromatic and/or apolar substances, like, e.g., a hydrophobic adsorbing resin, e.g. a high porosity hydrophobic adsorbing resin. This type of resin consists of microspheres of a diameter of 0.2 mm - 0.8 mm, with an uniformity coefficient ≤ 1.5 obtained by polymerization of Styrene and DVB without active groups, characterized by a highly porous physical structure having the parameter relative to the pore volume equal to about 1.3 ml/g enabling adsorption and selective elution of organic substances, preferably of aromatic nature. A non-binding example of resin useful for the carrying out of the process, e.g., consists of amberlite XAD-2, serdolit PAD-II, ADS TQ 318 or resins similar thereto.

The present invention therefore relates to a process for the preparation of a sage extract fraction comprising the following steps:

a. preparing a hydroalcoholic extract of sage leaves
b. obtaining from the extract prepared in a. a concentrated aqueous extract
c. decanting and/or filtering said aqueous extract collecting the supernatant or the filtrate, wherein said supernatant or filtrate comprises rosmarinic acid at a concentration in weight percentage of ≤3% and carnosol at a concentration in weight percentage of ≤0.6% and thujones in a percentage lower than or equal to 0.001%
d. the filtrate or the supernatant obtained in c. is subjected to one or more steps (passages) through a hydrophobic adsorbing resin and the eluate is collected

wherein said eluate thus obtained comprises rosmarinic acid at a concentration in weight percentage of ≤0.7%, carnosol at a concentration in weight percentage of ≤0.1% and thujones in a percentage lower than or equal to 0.001% and wherein said hydroalcoholic extract of step a. is prepared by subjecting sage leaves, optionally fragmented, to two or more extraction steps in ethanol at decreasing concentration ranging from 96% ethanol to 0% ethanol.

According to the present invention, step d. can be carried out on a column with resins as described above, able to adsorb aromatic or highly apolar substances and to let elute non-aromatic polar substances. The resin-adsorbed material, if desired, could be desorbed with a suitable solvent, like, e.g., ethanol or hydroalcoholic solvents, for other uses.

Suitable chromatography resins may be, e.g., high-porosity adsorption resins of styrene-divinyl benzene copolymer, like, e.g., amberlite XAD-2, serdolit PAD-II or the like. Therefore, the passage through resins having adsorbing features of the above-described type enables to obtain a fraction depleted in rosmarinic acid (≤0.7%), carnosic acid and carnosol (≤0.01%) and thujones that will be in a percentage lower than or equal to 0.001%.

The passage through resin could be repeated more than once and the extract fraction obtained could then be concentrated if desired.

The extract fraction obtained with the above-indicated processes will contain only traces also of the other substances having a phenolic structure present in the initial product, besides rosmarinic acid.

This further step d therefore enables a further elimination of rosmarinic acid and carnosic acid and derivatives thereof (carnosol), as the extract fraction obtained from the process comprising steps a, b, c, d comprises rosmarinic acid at a concentration in weight not greater than 0.7% ($\leq$0.7%), carnosic acid and carnosol at a concentration in weight not greater than 0.1% ($\leq$0.1%), in particular $\leq$0.05% and substantially free from thujones.

The extract fraction obtained in step d has features like those reported in

Table 1, where it is compared with the initial extract, with the extract obtained in c and with the retentate or eluate obtained in d and d', respectively.

Table 1

| Analysed sample | % Carnosic acid and carnosol | % Rosmarinic acid | % Thujones | % Mucoadhesion on an inclined plane | % barrier effect with IL assay |
|---|---|---|---|---|---|
| Reference hydroalcoholic extract | 0.52 | 5.5 | + | 58 | 28 |
| Extract fraction obtained in step d. | $\leq$0.05 | $\leq$0.7 | - | 39 | 50 |

The process according to the present invention, in the order of the steps as described above, enables therefore to obtain a fraction of sage extract as described above, which is depleted in rosmarinic acid, carnosic acid and derivatives thereof and thujones and surprisingly rich in substances having a protective action. The above-described process also eliminates resins via the reagents and steps used, as evident to the technician in the field.

Hence, the extract of the invention will also be substantially free from resins that will be found in discarded fractions.

As it may be seen from the above table, the fraction of the invention, obtainable with the above-described process, keeps a significant mucoadhesive ability in spite of the removal of resins, and exhibits a substantial increase of the barrier effect.

The authors of the present invention have in fact examined the mucoadhesive and barrier effect abilities of the whole (non-fractioned) sage extract, like, e.g. the hydroalcoholic extract of sage leaves, and have found that such extract did not possess a good mucoadhesive ability measurable in standard experiments for mucoadhesion measurement, like the assay of adhesion to mucin on an inclined plane and possessed a rather low barrier effect, measurable, e.g., by a simple assay reported in the experimental section of this description.

The authors of the present invention have found that a fraction, among those analysed, besides having a very low percentage of the active principles for which sage is commonly used, retained good desired mucoadhesive abilities and a barrier effect much greater than that of the initial extract.

The barrier effect of a compound on mucous membranes and skin may be measured in any way available to the technician in the field. For instance, the barrier effect may be measured by cell markers release inhibition assay in response to an inflammatory or irritating agent as described in detail in the experimental section below.

In short, however, an *in vitro* method for the assessment of the barrier effect of a sample on a cell culture can be summarized with the following steps:

a) preparing a cell culture;

b) placing the sample of interest (e.g. a fraction of sage extract or a non-fractioned extract of sage) on a semi-permeable membrane separating it from said culture, and, subsequently or concomitantly, adding to said sample a sample (e.g., an inflammatory agent such as bacterial lipopolysaccharide LPS or an interferon; an irritating or allergenic agent like ovoalbumin, dinitrochlorobenzene, oxazolone, eugenol, penicillin G, nickel sulphate) that, when in contact with said culture, be able to induce release of a marker (e.g., histamine or $\beta$-glucuronidase or a cytokine like IL-8, , IL-6 , IL1$\alpha$, TNF$\alpha$, TNF$\beta$) from said culture;

c) detecting in one or more successive time instants the concentration of said marker in the experiment prepared in a) and b) and comparing the measurements obtained relative to one or more reference values, wherein the lack of increase over time of said marker in step c. denotes a barrier effect of the sample (or even an increase of marker measurable only over a time longer than the control, in which the sample under examination is not placed on the semi-permeable membrane).

In particular, step c) can comprise:
detecting in one or more successive time instants the concentration of said marker in the experiments prepared in a) and b) and in a parallel control experiment in which the helichrysum extract fraction is not added on the permeable membrane in b) and comparing the measurements obtained from the two experiments in the same time instants.

Step c) could also comprise:
detecting in one or more successive time instants the concentration of said marker in the experiment prepared in steps a) and b) relative to the amount of said marker measured in the same experiment before step b).

In one embodiment the method can further comprise a further step of preparing an internal control in which cultured cells are pretreated with the substance adapted to induce marker release and the sage extract fraction is placed on the semi-permeable membrane in the absence of said substance, which could be added later to the fraction under examination in case said substance were to settle before said fraction.

According to one embodiment, the barrier effect is measured by IL-6 inhibition assay in response to an agent such as LPS.

$$\text{Barrier effect} = \%\ \text{reduction in release of IL-6 cytokine} = 100 - [(\text{pg/}\mu\text{L cytokines released from sample/pg/}\mu\text{L cytokines released from C+}) \times 100]$$

In an interesting embodiment, the sage extract fraction of the invention has a concentration in weight of rosmarinic acid of $\leq 0.7\%$, concentration in weight of carnosic acid and carnosol of $\leq 0.1\%$, in particular $\leq 0.05\%$; is substantially free from thujones and has a mucoadhesion ability, measurable with assay of mucoadhesion on an inclined plane (as described below) of 39% and a barrier effect equal to 50% in assay of inhibition of cell markers release in response to an inflammatory or irritating agent (like, e.g. inhibition of cytokines, such as IL-6 and/or IL-8, release as in response to LPS).

The present description also relates to a composition comprising the above-described extract fraction, useful in the protection of the skin or mucous membranes.

Said protection, of course with reference both to the fraction per se and to the composition, could be a preventive protection or a protection of partially compromised skin or mucous membranes.

For instance, the composition or the fraction of extract can, by virtue of their barrier effect, facilitate the repair of damaged skin with entailed restoration of healthy and elastic skin.

The skin lesions according to the present invention are, e.g. lesions that may involve also tissue underlying the skin and in which no open wounds are present.

By skin lesions not implying the presence of open wounds, according to the present description, are meant those lesions in which the superficial layer of the skin, and the underlying layers, though not being wounded, are particularly fragile, irritated and damaged.

Non-limiting examples of this type of lesions are represented by first-degree burns, first-degree decubitus lesions, pressure lesions, newly cicatrized rashes, wounds or burns, irritations, erythemas.

The composition or the fraction of extract of the invention could then be used for the treatment or the prevention of skin lesions not involving the presence of open wounds, or in the prevention or slowing down of worsenings of the same.

The compositions according to the invention could advantageously comprise further components, e.g. of plant origin, having, e.g. emollient, digestive, pro-kinetic, cholagogic, carminative, prebiotic, relaxing, excipient, preserving, wetting properties.

When the composition or the fraction of extract of the invention are used for the protection of the skin, the application thereof will be topical. The embodiments of the compositions for topical use are reported hereinafter in the description.

The composition according to the invention could be made, e.g., as oil-in-water emulsion, water-in-oil emulsion, oil-in-gel or gel-in-oil emulsion, multiple emulsions, sprays, and anhydrous formulations (pomade, gel, paste, cream, ointment) and will comprise one or more excipients suitable for the making of the desired end form.

Such excipients could be, e.g., emulsifying agents (cetearyl alcohol, cetearyl glucoside, hydrogenated castor oil) rheological additives, antioxidants (like, e.g., vitamins, tocopherols or other antioxidants known in the field).

The emulsifying agent could be a surfactant, which by lowering the interfacial tension decreases the free energy of the system; alternatively, also non-surfactant substances can be used, such as gum arabic, gelatin, hydrophilic colloids or finely subdivided powders (e.g., talc). In one embodiment, the excipients could be present at an overall concentration in weight comprised between 3 and 8%, such concentration being of course indicative, taking into account that anyhow the technician in the field will know how to adapt the concentration of the necessary excipients according to the embodiment he/she intends to prepare without addition of inventive activity.

Moreover, the composition could comprise perfuming and/or coloring agents which give it a pleasant smell, like, e.g., one or more essential oils like, e.g., lavender essential oil, melaleuca essential oil, lemon, mint, orange, and/or coloring agents which allow, e.g., to easily recognize the areas of application of the composition, the coloring being, e.g., temporary, so that it does not interfere with other later applications of the composition.

In one embodiment, said coloring and/or perfuming agents are comprised at an overall concentration in weight of from 0.001 to 3%.

By "overall concentration in weight" it is meant the concentration in weight in the composition of the sum of the various excipients, or the concentration in weight in the composition of the sum of the various perfuming and/or coloring agents present in the composition.

As to the use of the composition or of the fraction of extract in the protection of the skin, the invention also relates to medical devices like, e.g., medicated patches, medicated gauzes, medicated bandages, medicated towels, medicated pads, medicated diapers, i.e., patches, gauzes, bandages, towels, pads or diapers which comprise, or be at least partially covered by, or be at least partially imbued with the described composition of the invention in the most appropriate form.

The gauzes could be made as greasy gauzes, and so the bandages and the patches, using the composition made in the form, e.g., of an ointment, paste or cream. The towels could be imbued with the composition in the form of an oil emulsion with water or gel, and the diapers or the absorbent pads could be made by inserting the composition in the relevant layers known in the art for the insertion of protective or anti-irritating compositions.

Such products, like, e.g., infant diapers, absorbent pads (commonly called "sanitary pads") for women and adult incontinence pads, are commonly used, e.g., in infancy-, women- and geriatrics-related fields, and the technician in the field will know where to insert the composition described herein and which embodiment be the most suitable one without the need of particular teachings and only based on conventional techniques in the art.

Such devices will be applicable to the part to be treated and/or protected for a preventive purpose.

As already indicated above, the composition of the invention may be a composition for topical use which could be realized in the form of oil-in-water emulsion, water-in-oil emulsion, multiple emulsions, sprays, and anhydrous formulations (pomade, ointment, gel, paste, cream, spray) according to techniques commonly used in the field. The formulation indicated herein as "spray" could be an anhydrous formulation or even a formulation in emulsion, the form with an atomizer enabling also self-applications in zones harder to reach (like, e.g., the back), useful in all those cases in which the formulation is used, e.g., to alleviate sun rashes, erythemas, or skin irritations of various kind.

According to another embodiment, the extract fraction o the composition of the description could be used, thanks to their mucoadhesive and barrier effects, for the protection of the mucous membranes.

In this case as well, said protection could be a preventive protection, e.g. in all those cases envisaging an administration of drugs having the side effect of attacking the mucous membranes, or in those patients exhibiting conditions of recurring irritation of the same. In other cases, the protection could instead be a protection for curative purposes or in order to avoid the worsening of irritated or partially compromised mucous membranes.

In these cases, the administration of the extract or of the composition could be topical for all those mucous membranes on which a topical administration is possible (e.g., oral, rectal, vaginal, nasal mucosa) or oral in the cases in which said membranes be, e.g., intestinal or gastric mucosal membranes.

The compositions for the protection of the mucous membranes could therefore be made in the form of capsule, tablet, lozenge, granule, powder, syrup, elixir, hard gelatine, soft gelatine, suspension, emulsion, solution, suppository, cream, gel, spray, ointment, pomade, paste, oil-in-water emulsion, water-in-oil emulsion, oil-in-gel emulsion, gel-in-oil emulsion.

In case of formulations for topical use, the above indications related to the compositions for the protection of the skin could be followed, or syrups, rinses, sprays could be made, e.g., for the protection of the mucous membranes of the mouth, throat, nose.

For application on the rectal mucosa, suppositories or enemas or microenemas could be used, with the excipients known to the technician in the field for the making of such compositions.

As already mentioned, the composition herein described could be in the form of capsule, tablet, lozenge, hard gelatine, soft gelatine, granule, powder, syrup, elixir, suspension, emulsion. For oral administration the composition could be made in the form of daily unit dosages or of fractions of daily unit dosages (e.g. 2, 3, 4, 5, 6, or more capsules, tablets, lozenges, granule or powder single-doses, or gelatins could be taken over the day, in accordance with the judgment of the treating doctor), and may contain conventional excipients including, e.g., binding agents, like gum arabic, gelatine, sorbitol, gum tragacanth, and/or polyvinylpyrrolidone; fillers, like lactose, sugar, corn starch, rice starch, calcium phosphate, sorbitol and/or glycine; tableting lubricants, like magnesium stearate, talc, polyethylenglycol and/or silica; disintegrants, like potato starch; and wetting agents like sodium laurylsulphate. The tablets can be coated according to methods well-known in the standard pharmaceutical practice.

The composition could also be made in a liquid or semi-liquid form, as a suspension, emulsion, solution for oral administration and could optionally contain natural aromatizing agents giving a palatable taste thereto.

The composition in the form of powder or granule could be pre-metered in suitable containers and ready for use, either by ingestion as such or to be resuspended in an appropriate liquid such as water, tea, etc. In this case as well, the composition could contain natural aromatizing agents giving a palatable taste thereto.

Evidently, all of the above-indicated excipients could be used in a pharmaceutically acceptable grade.

In one embodiment, the composition as described herein, in any one of the above-indicated embodiments, could be in the form of pharmaceutical composition, i.e. comprise pharmaceutical-grade ingredientsor into a medical device.

The composition according to the present description could be made in the form of pharmaceutical composition or of medical device according to any one of the classes described in Directive 93/42/EEC on medical devices (comprising also substances and not only "devices" in the mechanical sense of the term), or in the form of medical food, food supplement, or in any form according to the regulatory provisions of the Country in which said composition will be produced.

The medical food herein described could also contain as ingredients other components, comprising e.g. combinations of vitamins, mineral salts and other substances directed at diet supplementing.

The extract fraction or the composition of the invention is therefore useful for its mucoadhesion and barrier effect properties in all those cases in which the protection of skin or mucous membranes is needed or desirable: those may be cases in which a drug that attacks mucous membranes or skin has to be administered, therefore in order to prevent or limit the damaging action of the drug, or in those cases in which the protection of a partially compromised skin or mucous membrane is preferable or desirable so as to enable a better and quicker healing thereof, defending it from further aggressions, or in those cases in which an individual has a chronic disorder in which skin or mucous membranes sustain irritations or alterations, therefore a barrier effect can prevent or limit damages on the skin or the mucous membrane.

Moreover, the extract fraction of the invention may be used as carrier for active principles in pharmaceutical compositions or into special-purpose foods (medical foods), as by virtue of its mucoadhesive properties it promotes adhesion thereof to mucous membranes.

Such fraction, therefore, can be formulated with active principles different from those of sage, and promote adhesion of such active principles to the mucous membranes.

The disclosure also relates to a method for the treatment or for the prevention the onset or the worsening of skin lesions not involving the presence of open wounds, wherein such method comprises one or more applications of the composition of the invention or of the medical device comprising it once or more per day on the concerned part.

The application of the composition, for instance, could be repeated whenever needed (e.g. at each change of pad in case of prevention of incontinence-related rashes) or once, twice, thrice, four or more times per day in general.

The invention also relates to a method for the preparation of a composition according to any one of the embodiments described above, wherein a sage extract fraction depleted in rosmarinic acid, carnosic acid and derivatives thereof (carnosol) and thujones wherein said rosmarinic acid has a concentration in weight percentage $\leq 3\%$, said carnosic acid and carnosol has a concentration in weight percentage of $\leq 0.6\%$ and said thujones are in a percentage lower than or equal to 0.001%, or also wherein said rosmarinic acid has a concentration expressed as percentage in weight $\leq 0.7\%$, said carnosic acid and carnosol has a concentration expressed as percentage in weight $\leq 0.1\%$ or 0.5% and said thujones are in a percentage lower than or equal to 0.001%, is admixed with at least one among excipients and/or substances of natural and/or plant origin (sage excluded) having emollient, digestive, pro-kinetic, cholagogic, carminative, prebiotic, relaxing, excipient, preserving, wetting properties as described above. In other embodiments, the composition according to the invention could comprise active principles, excluding those of sage, or could instead comprise only further substances of plant/natural origin having a barrier and/or mucoadhesive effect, substantially free from active principles.

Among these, e.g., could be used one or more of: gentian root extract, boldo leaves extract, milk thistle fruits extract, artichoke leaves extract, dandelion root extract, anise fruit extract, rosemary leaf extract, mint leaf extract, marjoram leaf extract, cumin fruit extract, coriander fruit extract, ginger root extract, fennel fruit extract, caraway fruit extract, charcoal, inulin.

Object of the present disclosure is also a method for the protective (preventive or curative) treatment of the skin or mucous membranes providing the administration of the extract fraction or the composition of the present invention to a patient in need thereof. Such administration could also be concomitantly with the administration of other drugs.

The absence of pharmacologically active principles of sage in the extract fraction makes such products particularly suitable to administration concomitantly with other drugs, as a side effect of interaction between the extract or the composition of the invention and the drug coadministered or concomitantly administered is markedly unlikely.

A non-limiting example of the method of treatment and/or of prevention of the skin or mucous membranes, could comprise the administration, subdivided into a single dose or plural doses, described of the fraction or the composition according to the present description, for a period of time of between one and six weeks, e.g. of between three and six weeks or even for a period of time higher than six weeks, in accordance with the judgment of the treating doctor.

Such administration could precede the administration of the drug even for a prolonged period, so as to optimize the health state of the skin or of the mucous membrane to be treated.

Alternatively, the administration could be concomitantly with the drug or with active principles having a pharmacological action.

The treating doctor will know how to establish both the most appropriate dosage and the administration times also on the basis of the patient's health state, weight, sex and age.

One of the substantial differences between the structure of the skin and that of the mucous membranes is represented by the absence, in the mucous membranes, of a selective barrier such as the corneous layer. Oral mucous membranes contact with noxious or irritating substances present in the environment (pollutants, pathogenic microorganisms, etc.) can therefore cause a high penetration of said substances both inside the mucous membranes and in the related airways (bronchi, lungs, etc.) causing inflammatory and/or allergic pathologies.

The protective action of the extract fraction of the present invention was assessed by mucoadhesion assays and barrier effect assays.

The former aim at evaluating the mucoadhesivity of the product under examination in order to establish whether such product has the ability to adhere to the mucous membranes and consequently exert a protective action.

For instance, a simple model is available for evaluating mucoadhesivity of the product of interest.

The model evaluates the mucoadhesive ability of the sample by measuring its ability to bond to mucin disposed on an inclined plane, allowing the moving away by sliding of the sample without mucoadhesive ability. For evaluation of the barrier effect an *in vitro* model is available enabling to evaluate the impediment of the sample to transit of LPS, accountable, in the model, for the freeing of mediators such as interleukin 6 (IL-6), measured semi-quantitatively by ELISA assay.

**Evaluation of adhesion percentage bv inclined plane with mucin.**

Measurements are performed by an equipment comprised of a 45°-inclined plane, thermostated to 37°C; below said plane a microbalance is set, which performs measurements at regular 1-s intervals and prints recorded measurements on paper. Performed measurements are then transcribed and reprocessed on Excel spreadsheet.

The inclined plane acts as support for the biological substrate, consisting of a mucin film set up by depositing, on a plexiglas plane kept horizontal, a volume equal to 3 ml of a suspension of porcine gastric mucin at an 8% concentration (w/w) in a phosphate buffer, pH 6.4.

The dispersion is then brought to dryness at the temperature of 44°C so as to obtain a film of known surface area equal to 33.6 cm$^2$.

An exactly weighted amount of the extract according to the invention is dissolved in a solvent. The extract is then deposited on the measurement plane with a multichannel pipette at constant time and rate during 6 seconds.

The multichannel pipette is calibrated so as to meter the solution (or dispersion) over the work plane. The total amount is measured at each assay (the weight of said volume is measured before the assay and depends on the density of the sample under analysis).

The sample, which will fall on the microbalance at the end of the run, is loaded in the top part of the inclined plane, containing the mucin film, and let slide thereon. The amount of fallen sample is measured by recording the weight variation as a function of time. The assay as made herein lasts 40 seconds, though usually after 20 seconds there is no weighing variation.

Measurements in the absence of the mucin film on the inclined plane are also performed (measurements indicated as blank) with the same amounts of sample and under the same assaying conditions.

Thus, it is possible to assess the intrinsic sliding properties of the sample, regardless of its ability to interact with the biological substrate.

The adhered % is calculated: defined as the amount of sample remained adhered to the mucin film or to the inclined plane (without mucin, blank) at the end of the measurement, normalized with respect to the amount deposited on the inclined plane.

Moreover, it is determined the percentage difference between sample amount adhered to the plane with mucin and sample amount adhered to the plane without mucin. The calculation is performed in accordance with the following equation:

$$\% \text{ difference} = (\text{adhered mucin } \% - \text{adhered blank } \%)/\text{adhered } \% \text{ of the blank}$$

where

- adhered mucin % = sample percentage remained adhered to the mucin film at the end of the measurement
- adhered blank % = sample percentage at the inclined plane at the end of the blank measurements.

**Barrier effect assay**

The barrier effect assay is a biological-type *in vitro* assay employed for assessing the ability of finished products and/or raw materials to protect, through the formation of a thin "insulating" layer, mucous membranes and skin from contact with environmental contaminants (dust, pollens, microorganisms, etc.)

The assay was developed to simulate *in vitro* the action exerted by products that are applied on skin and/or mucous membranes with the aim of creating a protective film against external aggressors.

The model takes advantage of the principle whereby cells subjected to contact with an inflammatory agent produce and secrete pro-inflammatory mediators (cytokines) in the extracellular environment in an amount related to the degree of inflammation caused; within a certain range, a direct proportionality exists between concentration and times of exposure to the inflammatory agent and amounts of released cytokines.

The experimental model adopted provides two chambers physically separated by a semi-permeable membrane (0.4 $\mu$m pores). Cells are seeded in the lower chamber, whereas the upper chamber accommodates the inflammatory agent; on the semi-permeable membrane separating the two chambers a thin film of the sample under analysis is stratified to highlight, if present, a barrier effect to the free transit of the inflammatory agent.

The semi-permeable membrane allows passage of the inflammatory agent in the lower chamber and constitutes the support on which the sample to be assayed is stratified. Depending on the "insulating" ability of the sample, a decrease of LPS migration from the upper chamber to the bottom one will be had (therefore a lesser stimulation of cells to cytokine production).

EXAMPLES

**Example 1**

**Preparation of extract fraction**

Sage leaves were fragmented and subjected to four steps of extraction in ethanol at decreasing concentrations; performing a step in 85% ethanol, a step in 50% ethanol, a step in 20% ethanol, a step in 5% ethanol.

The alcoholic extracts obtained as described were gathered in a 25:25:25:25 ratio and subjected to centrifugation for a time of 1-5 minutes at a rotation rate equal to 3500 rpm; the supernatant was recovered, and concentrated by ethanol evaporation with use of a thin film distillation system according to the standard protocol, providing the feeding of the extract to be concentrated at a rate of about 500 l/h; operation was by setting a residual vacuum of 0.6-0.8 bars and the fluid heating the evaporation walls was set at 140°C, thereby obtaining, after ethanol elimination, a concentrated aqueous extract.

The aqueous extract thus obtained was filtered on a panel filter with a cutoff of 25 micrometers.

The filtrate thus obtained, which comprised rosmarinic acid at a concentration in weight not greater than 3% and a concentration in weight of carnosic acid and derivatives thereof not greater than 0.6% and thujones in traces, was subjected to one or more steps with a high porosity adsorbing resin, in particular a resin of styrene-DVB copolymer of ADS TQ 318 type was used.

The remaining active principles are retained by the resin and the eluate is collected.

By this process, it is obtained an extract comprising rosmarinic acid at a concentration in weight not greater than 0.7% and carnosic acid and derivatives thereof at a concentration not greater than 0.1% and traces of thujones.

In this case a standard process of obtainment of the extract by treatment on resin entails the introduction of the concentrated aqueous extract into a chromatographic column containing the adsorption resin. The feeding fluid must have suspended solids indicatively comprised between 0.2 to 1° Bix, the feed rate is comprised between 1 and 4 BV/hour, and preferably 2 BV/hour. The corresponding aqueous eluate is collected and subjected to drying by lyophilization, or to desiccation; the resin-adsorbed substances are eluted by using 96% ethyl alcohol, or methanol or acetone, preferably 96% ethyl alcohol. In this latter case, the alcoholic eluate is subjected to desiccation or to lyophilization (after having added, in this latter case, water in a 1:1 v/v ratio with the alcoholic eluate and having evaporated the ethyl alcohol), useful for other purposes.

**Example 2**

**Assessment of mucoadhesive ability of the extract of the invention on an inclined plane**

Measurements were performed by means of an equipment comprised of a 45°-inclined plane, thermostated to 37°C; below said plane a microbalance is set, which performs measurements at regular 1-s intervals and prints recorded measurements on paper. Performed measurements were transcribed and reprocessed on Excel spreadsheet.

The inclined plane acts as support for the biological substrate, consisting of a mucin film. The mucin film was set up by depositing, on the plane consisting of plexiglas kept horizontal, a volume equal to 3 ml of a suspension of porcine gastric mucin at an 8% concentration (w/w) in a phosphate buffer, pH 6.4.

The dispersion was brought to dryness at the temperature of 44°C so as to obtain a film of known surface area equal to 33.6 cm$^2$. Amount, concentration and deposition plane were defined during process development.

An exactly weighted amount of the extract of the invention was dissolved in solvent (1% water). The extract fraction was deposited on the measurement plane with a multichannel pipette at constant time and rate during 6 seconds.

The multichannel pipette was adjusted so as to meter 280 microliters of solution (or dispersion) over the work plane. The total amount measured at each assay is of 1.12 ml per sample (the weight of said volume is measured before the assay and depends on the density of the sample under analysis).

The sample, which would fall on the microbalance, was loaded in the top part of the inclined plane, containing the mucin film, and let slide thereon.

The amount of fallen sample was measured by recording the weight variation as a function of time.

The assay lasted 40 seconds, however it is stressed that after 20 seconds there was no weighing variation.

Measurements in the absence of the mucin film were also performed (measurements indicated as blank) with the same amounts of sample and under the same assaying conditions, but without the mucin layer deposited on the plane.

Thus, it is possible to assess the intrinsic sliding properties of the sample, regardless of its ability to interact with the biological substrate.

The adhered % is calculated: defined as the amount of sample remained adhered to the mucin film or to the inclined plane (without mucin, blank) at the end of the measurement, normalized with respect to the amount deposited on the inclined plane.

Moreover, it is determined the percentage difference between sample amount adhered to the plane with mucin and sample amount adhered to the plane without mucin. The calculation is performed in accordance with the following equation:

$$\% \text{ difference} = ( \text{adhered mucin } \% - \text{adhered blank } \%) / \text{adhered } \% \text{ of the blank.}$$

where

- adhered mucin %= sample percentage remained adhered to the mucin film at the end of the measurement
- adhered blank % = sample percentage at the inclined plane at the end of the blank measurements.

Analysed samples:
The measurements were performed for the following samples:

- water: used as negative control, i.e. as a solution without mucoadhesion
- 0.7% sodium alginate in water used as positive control, i.e. as a substance with known mucoadhesive properties
- Sage sample: lyophilized sage fraction, 1% solution in water.

**Results and discussion**

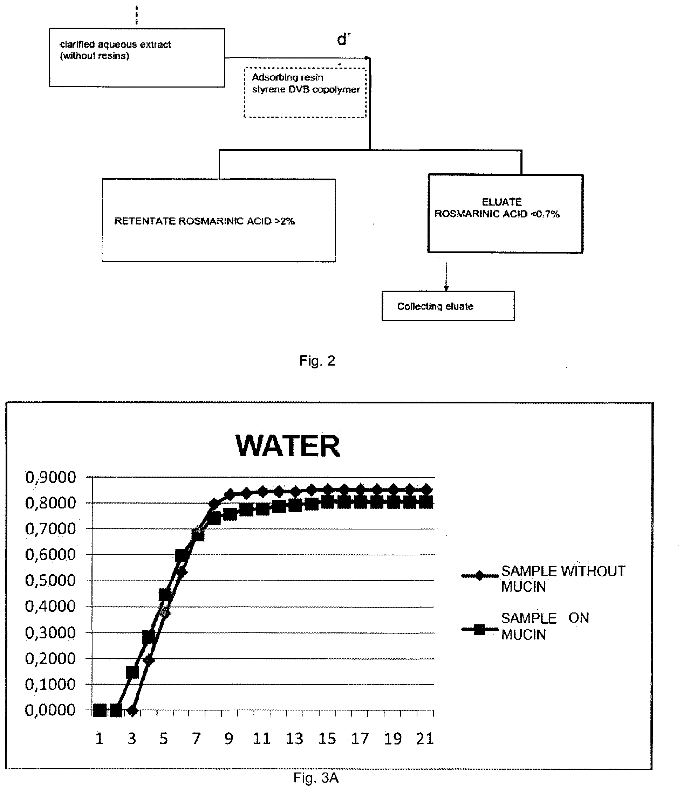

In the graphs reported in Figures 3A, B and C are represented, respectively, the sliding profiles obtained with the negative control (water), the positive control (0.7% sodium alginate) and the sample under analysis (sage, 1% solution in water).

From the figures, it is evident how the slid amount in the presence of mucin be at each time definitely lower than that fallen in blank measurements, to indicate occurred interaction between the formulation and the biological substrate. Said difference remains at the end of the measurement.

The figures also demonstrate that, despite the elimination of resins (eliminated along with rosmarinic acid and carnosic acid and derivatives thereof), the sage extract according to the present invention, even at a 1% dilution, shows am mucoadhesive ability greater than that of the solvent.

In the table below, mucoadhesion parameters are reported, measured as described in the experimental section.

Table 2

| | % DIFFERENCE (between percentage adhered to the inclined plane with mucin and without mucin |
|---|---|
| Extract object of the invention, obtained in d (% adhered to the plane with mucin -% adhered to the plane without mucin)/ % adhered to the plane without mucin) | 39% |
| 0.7% SODIUM ALGINATE, (% adhered to the plane with mucin - % adhered to the plane without mucin)/ % adhered to the plane without mucin) | 118% |
| Water %, (% adhered to the plane with mucin -% adhered to the plane without mucin)/ % adhered to the plane without mucin) | 23% |
| Reference hydroalcoholic sage extract | 58% |

## EXAMPLE 3.

### Barrier effect assay

For the barrier effect assay, two chambers physically separated by a semi-permeable membrane ($0.4\mu m$ pores) were used. Human fibroblast cells were seeded in the lower chamber, whereas an inflammatory agent, LPS (purified E. Coli lipopolysaccharide) was introduced into the upper chamber; on the semi-permeable membrane separating the two chambers a thin film of sage extract was stratified as described in the present disclosure.

Induced inflammatory response was estimated through semi-quantitative dosage of Interleukin-6 (IL-6) released in the culture medium of the lower chamber: Barrier effect assessment was obtained by comparison with the positive control in which the two chambers were separated by the same type of semi-permeable membrane, free however from any barrier.

The greater the barrier effect exerted by the stratified film of substance, the lesser the inflammatory action observed in the cells present in the chamber therebelow, as the inflammatory agent in the chamber thereabove will have greater difficulty to cross the semi-permeable chamber.

As to the threshold value above which it may be said that a substance causes a barrier effect in the assay reported herein, a value equal to 15% of inhibition as compared to the control was identified by the Inventors, during the setting up of the assay, on the basis of assays performed on substances known to have a barrier effect.

Then the barrier effect of the extract fraction as described herein was evaluated.

The data reported below show how the fraction has a remarkable barrier effect.

The assessment assay was carried out as follows through a method developed to simulate *in vitro* the protective action of substances and formulations which, when applied to the skin and mucous membranes *in vivo,* form an "insulating" effect against environmental agents.

The model takes advantage of the principle whereby cells subjected to contact with an inflammatory agent produce and secrete pro-inflammatory mediators (cytokines) in the extracellular environment in an amount related to the degree of inflammation caused. The greater the amount of inflammatory agent reaching the cells, the greater the amount of cytokines released.

The model envisages the arrangement of two chambers physically separated by a semi-permeable membrane which allows the passage of sufficiently small-sized solutes.

In the lower chamber, consisting of a well containing plates for cell cultures, HuDe cells (no. BS PRC 41, bought from the Istituto Zooprofilattico di Brescia, Italy) are grown, while the upper chamber, consisting of an insert for complex cell cultures (transwells), accommodates the inflammatory agent.

On the surface of the semi-permeable membrane of the insert separating the two chambers, before the introduction of the inflammatory agent in the upper chamber, a thin film of the sample being examined is stratified to assess any BE upon the free passage of the inflammatory agent.

As a function of the insulating capabilities of the sample, there will be had a decrease of the migration of the inflammatory agent from the upper chamber and, as a consequence, a lesser stimulation of the cells to produce cytokines. The extent of the inflammatory reaction is estimated through the semi-quantitative dosage of cytokines released in the culture medium of the lower chamber, in particular of interleukin 6 (IL-6).

As a control a similar experiment is used in which no sample is stratified on the membrane, thus making it possible to measure the effect of the inflammatory agent without any barrier in addition to the semi-permeable membrane.

Further, an internal control is used in which the cultured cells are pre-treated with the substance adapted to induce the release of the marker and the sample is placed on the semi-permeable membrane in the absence of said substance, one or more measurements in time are thus performed of the quantity of marker in the culture medium of said internal control. In the internal control, the cells are therefore stimulated first with the inducing substance and then there has to be assessed whether the sample which may pass the membrane and go into the cells pushed by the medium above has any effect in decreasing the release of the marker not related to the barrier effect. For instance, when an inflammatory agent is used as the inducing substance, the internal control makes it possible to understand if the reduction in the concentration of cytokines in the culture medium is due to the barrier effect or if the sample that may pass into the cells pushed by the medium above has any effect in reducing the inflammatory response independently of the barrier effect.

The barrier effect (BE) is expressed as a percentage of the reduction of the release of IL-6 and is calculated through comparison with the positive control in which the two chambers are separated by the same type of semi-permeable membrane without the barrier created by the sample.

3.1 PREPARATION OF THE CELL CULTURE:

For each assayed sample, cells of the HuDe cell line were seeded in the wells of a cell culture plate, one for the barrier assay (BA) and another one for the internal control at a density of 40,000 cells /ml in MEM medium supplemented with 10% bovine serum (FBS); 1 ml of cell suspension per well.

The cells are treated with the SAMPLE (CAM), with the POSITIVE CONTROL (C+) (inflammatory agent without sample), and with the NEGATIVE CONTROL (C-) (medium only) and each assay is carried out in triplicate.

The plates were incubated at 37°C, overnight (22-24 hours).

3.2 PREPARATION OF INSERTS FOR COMPLEX CELL CULTURES

Inserts for complex cell cultures (Becton Dickinson) are placed on other plates, and on each of them a fixed amount of collagen of 0.1 ml/ml is supplied. The plates were incubated at 37°C, overnight (22-24 hours).

**DAY 2**

3.3 VERIFICATION OF STATE AND LEVEL OF CELL CONFLUENCY

In order to proceed with the experiment, a confluency not lower than 95% is required.

3.4 COLLAGEN LAYER DRYING

From the two plates (BA and IC) with the inserts the collagen is removed and the insert left under the flow of the hood for the time required to let them dry completely (10÷15 minutes)

3.5 BARRIER assay (BA)

The steps described below are carried out in the culture plate for the BA

Setting up of the sample laver in the BA:

On the sample's semi-permeable membrane 100µl of a 2% sage extract-based composition according to the invention were inoculated and allowed to stratify for 20 minutes while in the C+ and C- inserts nothing is added. Once the 20 minutes have passed the excess sample is eliminated and the membranes are washed with PBS according to procedures specified by the protocol.

Addition of LPS (inflammatory agent) to BA inserts

Once the sample layer has dried, in the first three CAM inserts and in the three C+ ones, 300µl of the LPS (membrane lipopolysaccharide) were inoculated at the concentration of 1µg/ml while in the remaining three of the C- 300 µl of MEM medium with 5% FBS were added. The inserts are inserted in their respective wells with the cells and the plates are incubated for 1 h at 37°C and under an atmosphere enriched with 5% $CO_2$ overnight (22-24 hours).

Once the 1 h incubation is completed, the inserts are removed and discarded and the plates are incubated again overnight (22-24 hours).

3.6 INTERNAL CONTROL (IC) ASSAY:

The internal control assay was carried out concomitantly with the BA

Exposure of IC cells to LPS:

Once dried, in the first six inserts of IC, three for the sample to be analysed and three for the C+, 300µl of the LPS solution are inoculated while in the remaining three of the C- 300µl of the medium are added.
The inserts with LPS and MEM are then inserted in the wells with IC cells and all incubated for 1 h.

LPS removal and IC membrane drying:

Once the 1 h incubation is completed, the inserts are removed from the wells with the cells and transferred to the empty plate while the plate with the cells is placed in an incubator.
The LPS solution still present is removed from the inserts, the latter are subjected to a rapid wash with ultrapure sterile water and allowed to dry.

Arrangement of sample layer in the IC:

On the semi-permeable membrane of the three inserts for the sample 100µl of a 2% sage extract-based composition according to the invention were inoculated and allowed to stratify for 20 minutes while in the C+ and C- inserts nothing is added. Once the 20 minutes have passed the excess sample is eliminated and the membranes are washed with PBS according to procedures specified by the protocol.

Addition of LPS to IC inserts

Once the inserts with the sample are ready, 300 µl of medium are added to all inserts (CAM, C+, C-). The inserts are inserted in their respective wells with the cells and the plates are incubated for 1 h at 37°C.
Once the 1 h incubation is completed, the inserts are removed and discarded and the plates incubated again overnight (22-24 hours).

DAY 3

4.7 SUPERNATANT COLLECTION AND ENZYME IMMUNOASSAY

Once the 22-24 hours have passed, the supernatants are collected from the BA and the IC plates for performing the ELISA assay and semi-quantitative dosage of IL-6.

BARRIER EFFECT (BE) ASSESSMENT

The BE of a substance or compound is expressed as *% reduction in the release of IL-6 cytokine* by cells exposed to LPS wherein the sample has been assayed relative to the positive control (C+) wherein the cells have only been exposed to LPS.

$$\text{BE} = \% \text{ reduction in release of IL-6 cytokine} = 100 - [(\text{pg/µL cytokines released from sample}/\text{pg/µL cytokines released from C+}) \times 100]$$

Table 1, reported below, shows that the sage extract obtained according to the present invention creates a barrier to passage of LPS.

| SAMPLE | % INHIBITION OF IL-6 |
|---|---|
| Internal control | 0 |
| Extract fraction obtained according to the present invention | 50 |
| Reference hydroalcoholic sage extract | 28 |

As it may be seen from the table, the 2.0 % extract fraction of the invention exhibits a barrier effect distinctly greater that the initial extract and retains, in spite of resin removal a good mucoadhesive ability making it suitable for the uses claimed and described in the present application.

**Claims**

1.  A sage extract fraction depleted in rosmarinic acid, carnosic acid and carnosol and in thujones, wherein said rosmarinic acid has a concentration in weight percentage of ≤3%, said carnosic acid and carnosol have a concentration in weight percentage of ≤0.1% and said thujones are in a percentage lower than or equal to 0.001%, **characterized in that** it has a barrier effect greater than that of a non-fractioned hydroalcoholic extract of sage leaves.

2.  The sage extract fraction according to claim 1, wherein said fraction is **characterized by** a percentage of barrier effect, measured by cell marker release inhibition assay in response to an inflammatory or irritating agent, greater than or equal to 40% or greater than or equal to 50%.

3.  The sage extract fraction according to any one of claims 1 or 2 further depleted in resins.

4.  The sage extract fraction according to any one of claims 1 to 3 for use in the treatment or prevention of skin lesions which do not involve the presence of open wounds.

5.  A process for the preparation of a sage extract fraction, comprising the following steps:

    a. preparing a hydroalcoholic extract of sage leaves
    b. obtaining from the extract prepared in a. a concentrated aqueous extract
    c. decanting and/or filtering said aqueous extract collecting the supernatant or the filtrate, wherein said supernatant or filtrate comprises rosmarinic acid at a concentration in weight percentage of ≤3%, carnosic acid and carnosol at a concentration in weight percentage of ≤0.6% and thujones in a percentage lower than or equal to 0.001%,
    d. the supernatant or filtrate obtained in c. is subjected to one or more steps through a hydrophobic adsorbing resin and the eluate is collected wherein said eluate thus obtained comprises rosmarinic acid at a concentration in weight percentage of ≤0.7% and carnosic acid and carnosol at a concentration in weight percentage of ≤0.1% and said thujones are in a percentage lower than or equal to 0.001% and
    wherein said hydroalcoholic extract of step a. is prepared by subjecting sage leaves, optionally fragmented, to two or more extraction steps in ethanol at decreasing concentration ranging from about 96% ethanol to about 0% ethanol.

6.  The process according to claim 5, wherein said two or more steps in a. comprise a step in 85% ethanol, a step in 50% ethanol, and a step in 20% ethanol.

7.  The process according to claim 6, further comprising a step in 5% ethanol.

8.  The process according to any one of claims 5 to 7, wherein said passage through resin is carried out on a styrene-DVB copolymer high porosity adsorbing resin.

9.  A composition comprising the sage extract fraction according to any one of claims 1 to 3.

10. The composition according to claim 9 for use in the treatment or the prevention of skin lesions which do not involve the presence of open wounds.

11. The composition for use according to claim 10 in the form of suspension, emulsion, solution, suppository, cream, gel, spray, ointment, pomade, paste, oil-in-water emulsion, water-in-oil emulsion, oil-in-gel emulsion , gel-in-oil emulsion.

12. The composition for use according to any one of claims 10 or 11, wherein said composition is or is comprised in a pharmaceutical composition, is comprised in or consists of a medical device.

13. The composition according to claim 9 in the form of a medical device, drug, or medical food.

**14.** A pharmaceutical carrier comprising the extract fraction according to any one of claims 1 to 3, **characterized in that** it promotes the adhesion of the active principles to the mucous membranes.

**Patentansprüche**

**1.** Salbeiextraktfraktion, die in Rosmarinsäure, Carnosolsäure und Carnosol und in Thujonen verdünnt ist, wobei die Rosmarinsäure über eine Konzentration von $\leq 3$ Gewichtsprozent, die Carnosolsäure und Carnosol über eine Konzentration von $\leq 0{,}1$ Gewichtsprozent und die Thujonen über einen Prozentanteil von $\leq 0{,}001$ % verfügen, **dadurch gekennzeichnet, dass** sie über eine Barrierewirkung verfügt, die größer als die eines nicht fraktionierten Wasser-Alkohol-Extrakts von Salbeiblättern ist.

**2.** Salbeiextraktfraktion gemäß Anspruch 1, wobei die Fraktion durch einen Prozentsatz einer Barrierewirkung, gemessen mit einem Zellenmarkerfreisetzungshemmungsassay in Reaktion auf einen entzündlichen oder reizenden Wirkstoff, gekennzeichnet ist, der größer oder gleich 40 % oder größer oder gleich 50 % ist.

**3.** Salbeiextraktfraktion gemäß einem der Ansprüche 1 oder 2, die weiterhin in Harzen verdünnt ist.

**4.** Salbeiextraktfraktion gemäß einem der Ansprüche 1 bis 3, zur Verwendung bei der Behandlung oder Prävention von Hautverletzungen, die keine offenen Wunden umfassen.

**5.** Verfahren zur Herstellung einer Salbeiextraktfraktion, das die folgenden Schritte umfasst:

a. Herstellen eines Wasser-Alkohol-Extrakts von Salbeiblättern
b. Erhalten, aus dem in a. hergestellten Extrakt, eines konzentrierten wässrigen Extrakts
c. Dekantieren und/oder Filtern des wässrigen Extrakts, wodurch der Überstand oder das Filtrat gewonnen wird, wobei der Überstand oder das Filtrat umfassen: Rosmarinsäure mit einer Konzentration von $\leq 3$ Gewichtsprozent, Carnosolsäure und Carnosol mit einer Konzentration von $\leq 0{,}6$ Gewichtsprozent und Thujonen mit einer Konzentration von $\leq 0{,}001$ %.
d. Der Überstand oder das Filtrat, die in c. erhalten werden, werden in einem oder mehreren Schritten einem hydrophoben adsorbierenden Harz ausgesetzt und das Eluat wird gewonnen, wobei das so gewonnene Eluat umfasst: Rosmarinsäure mit einer Konzentration von $\leq 0{,}7$ Gewichtsprozent und Carnosolsäure und Carnosol mit einer Konzentration von $\leq 0{,}1$ Gewichtsprozent und dass die Thujonen über einen Prozentsatz von $\leq 0{,}001$ % verfügen, und
wobei der Wasser-Alkohol-Extrakt von Schritt a. dadurch hergestellt wird, dass Salbeiblätter, optional fragmentiert, zwei oder mehreren Extraktionsschritten in Ethanol bei abnehmender Konzentration von ungefähr 96 % Ethanol bis ungefähr 0 % Ethanol unterzogen werden.

**6.** Verfahren gemäß Anspruch 5, wobei die zwei oder mehr Schritte in a. umfassen: einen Schritt in 85 % Ethanol, einen Schritt in 50 % Ethanol und einen Schritt in 20 % Ethanol.

**7.** Verfahren gemäß Anspruch 6, das weiterhin einen Schritt in 5 % Ethanol umfasst.

**8.** Verfahren gemäß einem der Ansprüche 5 bis 7, wobei die Passage durch Harz auf einem Styrol-DVB-Copolymer-adsorbierendem Harz hoher Porosität ausgeführt wird.

**9.** Zusammensetzung, die die Salbeiextraktfraktion gemäß einem der Ansprüche 1 bis 3 umfasst.

**10.** Zusammensetzung gemäß Anspruch 9 zur Verwendung bei der Behandlung oder Prävention von Hautverletzungen, die keine offenen Wunden umfassen.

**11.** Zusammensetzung zur Verwendung gemäß Anspruch 10 in der Form einer Suspension, einer Emulsion, einer Lösung, eines Zäpfchens, einer Creme, eines Gels, eines Sprays, einer Salbe, einer Pomade, einer Paste, einer Öl-in-Wasser-Emulsion, einer Wasser-in-Öl-Emulsion, einer Öl-in-Gel-Emulsion, einer Gel-in-Öl-Emulsion.

**12.** Zusammensetzung zur Verwendung gemäß einem der Ansprüche 10 oder 11, wobei die Zusammensetzung eine pharmazeutische Zusammensetzung ist, oder darin enthalten ist, ein Medizinprodukt ist, oder darin enthalten ist.

**13.** Zusammensetzung gemäß Anspruch 9 in der Form eines Medizinprodukts, eines Arzneimittels oder eines medizinischen Nahrungsmittels.

**14.** Pharmazeutischer Träger, der umfasst: die Extraktfraktion gemäß einem der Ansprüche 1 bis 3, **dadurch gekennzeichnet, dass** er die Adhäsion der Wirkstoffe an die Schleimhäute fördert.

**Revendications**

**1.** Fraction d'extrait de sauge appauvrie en acide rosmarinique, acide carnosique et carnosol et en thuyones, dans laquelle ledit acide rosmarinique a une concentration en pourcentage en poids ≤ 3 %, lesdits acide carnosique et carnosol ont une concentration en pourcentage en poids ≤ 0,1 % et lesdites thuyones sont présentes à un pourcentage inférieur ou égal à 0,001 %, **caractérisée en ce qu'**elle a un effet de barrière supérieur à celui d'un extrait hydroalcoolique non fractionné de feuilles de sauge.

**2.** Fraction d'extrait de sauge selon la revendication 1, dans laquelle ladite fraction est **caractérisée par** un pourcentage d'effet de barrière, mesuré par un dosage d'inhibition de la libération des marqueurs cellulaires en réponse à un agent inflammatoire ou irritant, supérieur ou égal à 40 % ou supérieur ou égal à 50 %.

**3.** Fraction d'extrait de sauge selon l'une quelconque des revendications 1 ou 2 appauvrie en outre en résines.

**4.** Fraction d'extrait de sauge selon l'une quelconque des revendications 1 à 3 destinée à être utilisée dans le traitement ou la prévention des lésions cutanées qui n'impliquent pas la présence de plaies ouvertes.

**5.** Procédé de préparation d'une fraction d'extrait de sauge, comprenant les étapes suivantes :

a. préparation d'un extrait hydroalcoolique de feuilles de sauge
b. obtention à partir de l'extrait préparé en a. d'un extrait aqueux concentré
c. décantation et/ou filtration dudit extrait aqueux par collecte du surnageant ou du filtrat, où ledit surnageant ou filtrat comprend de l'acide rosmarinique à une concentration en pourcentage en poids ≤ 3 %, de l'acide carnosique et du carnosol à une concentration en pourcentage en poids ≤ 0,6 % et des thuyones en un pourcentage inférieur ou égal à 0,001 %,
d. soumission du surnageant ou du filtrat obtenu en c. à une ou plusieurs étapes de passage sur une résine d'adsorption hydrophobe et de collecte de l'éluat où l'éluat ainsi obtenu comprend de l'acide rosmarinique à une concentration en pourcentage en poids ≤ 0,7 % et de l'acide carnosique et du carnosol à une concentration en pourcentage en poids ≤ 0,1 % et lesdites thuyones sont présentes à un pourcentage inférieur ou égal à 0,001 % et
dans lequel ledit extrait hydroalcoolique de l'étape a. est préparé par soumission de feuilles de sauge, éventuellement fragmentées, à deux étapes d'extraction ou plus dans l'éthanol à une concentration décroissante allant d'environ 96 % d'éthanol à environ 0 % d'éthanol.

**6.** Procédé selon la revendication 5, dans lequel lesdites deux étapes ou plus en a. comprennent une étape dans 85 % d'éthanol, une étape dans 50 % d'éthanol, et une étape dans 20 % d'éthanol.

**7.** Procédé selon la revendication 6, comprenant en outre une étape dans 5 % d'éthanol.

**8.** Procédé selon l'une quelconque des revendications 5 à 7, dans lequel ledit passage sur résine est mis en oeuvre sur une résine adsorbante à haute porosité de type copolymère styrène-DVB.

**9.** Composition comprenant la fraction d'extrait de sauge selon l'une quelconque des revendications 1 à 3.

**10.** Composition selon la revendication 9 destinée à être utilisée dans le traitement ou la prévention des lésions cutanées qui n'impliquent pas la présence de plaies ouvertes.

**11.** Composition destinée à être utilisée selon la revendication 10 sous forme de suspension, émulsion, solution, suppositoire, crème, gel, spray, pommade, pâte, émulsion huile dans l'eau, émulsion eau dans l'huile, émulsion huile dans gel, émulsion gel dans l'huile.

**12.** Composition destinée à être utilisée selon l'une quelconque des revendications 10 ou 11, dans laquelle ladite composition est ou est contenue dans une composition pharmaceutique, est contenue dans ou consiste en un dispositif médical.

**13.** Composition selon la revendication 9 sous forme de dispositif médical, médicament, ou alicament.

**14.** Véhicule à usage pharmaceutique comprenant la fraction d'extrait selon l'une quelconque des revendications 1 à 3, **caractérisé en ce qu'**il favorise l'adhérence des principes actifs aux membranes muqueuses.

SAGE

a.

b.

1st alcoholic extract
85% EtOH

2nd alcoholic extract
50% EtOH

3rd alcoholic extract
20% EtOH

4th alcoholic extract
5% EtOH

admixing
extract
-decanting
-centrifuging

FRACTION 1
PRECIPITATE

CLARIFIED
ALCOHOLIC
EXTRACT

RESIN
ELIMINATION
tujone, carnosol

c.

-concentrating
-decanting
-centrifuging

EXHAUSTED
SAGE

Clarified CONCENTRATED
aqueous extract

FRACTION 2
PRECIPITATE

d.

optionally d'

colletcting retentate and/or eluate

e.

Fig. 1

Fig. 2

Fig. 3A

**0.7% SODIUM ALGINATE IN WATER**

Fig. 3B

**0.1% SAGE IN WATER**

Fig. 3C